# EUROPEAN PATENT APPLICATION

(11) **EP 4 410 959 A1**
(43) Date of publication of application: **07.08.2024**
(21) Application number: 22872587.5
(22) Date of filing: 10.08.2022
(51) Int. Cl.: C12N 5/071, A23L 13/00, C12N 5/077

(54) **CELL CULTURE GEL, METHOD FOR PRODUCING CELL CULTURE GEL WITH CELLS, METHOD FOR PRODUCING CELLS, THREE-DIMENSIONAL MUSCLE TISSUE PRODUCED BY SAME, AND CULTURED MEAT**

(30) Priority: 27.09.2021 JP 2021156532
(71) Applicant: The University of Tokyo, Bunkyo-ku, Tokyo 113-8654 (JP); NISSIN FOODS HOLDINGS CO., LTD., Yodogawa-ku Osaka-shi, Osaka 532-8524 (JP)
(72) Inventor: TAKEUCHI, Shoji, Tokyo 113-8654 (JP); MORIMOTO, Yuya, Tokyo 113-8654 (JP); FURUHASHI, Mai, Osaka-shi, Osaka 532-8524 (JP)
(74) Representative: Mewburn Ellis LLP
(86) International application number: PCT/JP2022/030607
(87) International publication number: WO 2023/047831

(57) **Abstract**

The present invention addresses the problem of providing, as a scaffold material, a cell culture gel that utilizes plasma from adult bovine blood, rather than using extracellular matrix. The present invention further addresses the problem of producing cells using, as the scaffold material for cell culture, a cell culture gel that utilizes plasma from adult bovine blood. The cell culture gel according to the present invention contains: plasma derived from adult bovine blood, and a coagulant.

## Description

### TECHNICAL FIELD

The present invention relates to a cell culture gel, a method for producing a cell-containing cell culture gel, a method for producing cells, three-dimensional muscle tissue, and cultured meat. In particular, the present invention relates to a cell culture gel utilizing plasma derived from adult bovine blood.

### BACKGROUND ART

The demand for meat has been accelerating worldwide accompanying global population growth, economic development in emerging countries, and the diversification of dietary habits. However, livestock production entails the raising costs, and, since livestock cannot be produced at unlimited levels, it is predicted that the supply of meat does not eventually catch up with the demand.
Research into the development of meat substitutes is thus being carried out as one solution for a stable meat supply.

When it comes to meat substitutes for meat produced from livestock, there are various types of them, and these may be classified into imitation meats, plant-based meats, cultured minced meats, cultured steak meats, and so forth. Among these, cultured minced meats and cultured steak meats are referred to as cultured meats; however, cultured minced meat is an aggregate of disconnected muscle cells and has an inferior texture as meat. In contrast to this, cultured steak meat reproduces the three-dimensional structure of muscle tissue and is a meat substitute that supports the perception of a meat texture.

In this context, cells have to be cultured three dimensionally in order to reproduce the three-dimensional structure of muscle tissue, that is, in order to construct three-dimensional tissue in vitro. However, there are many technical problems to be overcome in order to culture cells three dimensionally, and the following, for example, have to be investigated: how to fuse cells three dimensionally; how to construct the culture media, e.g., serum, scaffold material, and so forth; and can these materials be supplied in quantities and at costs that can support application to industrial production?

The proliferation of cells adhered to a scaffold material is basically required for three-dimensional cell culture. The following, for example, have previously been used as a scaffold material: treated extracellular matrix (ECM), purified extracellular matrix, and materials constructed to imitate extracellular matrix (refer, inter alia, to PTL 1).

However, the extracellular matrix is an insoluble material that is present outside the cells in an organism, and as a consequence separation from cells and purification have been required in order to use the extracellular matrix as a scaffold material in cell culture. In addition, there has been a problem in that extracellular matrix suitable for food applications is unknown and application to cases in which the three-dimensional tissue is cultured meat has not been possible.

### CITATION LIST

### PATENT LITERATURE

[PTL 1] Republished International Patent Application No. WO 2019/208831

### SUMMARY OF INVENTION

### TECHNICAL PROBLEM TO BE SOLVED BY INVENTION

The present invention addresses the problem of providing, as a scaffold material, a cell culture gel that utilizes plasma derived from adult bovine blood, rather than using extracellular matrix. The present invention further addresses the problem of producing cells using, as the scaffold material for cell culture, a cell culture gel that utilizes plasma derived from adult bovine blood.

### SOLUTION TO PROBLEM

As a result of intensive investigations in view of the circumstances described in the preceding, the present inventors have discovered a cell culture gel that utilizes plasma derived from adult bovine blood. The present inventors have also discovered a method for obtaining cells by carrying out cell culture using this cell culture gel.

That is, a cell culture gel according to the present invention is characterized in including: adult bovine blood-derived plasma; and a coagulant.

This adult bovine blood-derived plasma may be a platelet-rich plasma.

The coagulant for cell culture may be calcium chloride.

The cell culture gel may further include a culture media component.

A method according to the present invention for producing a cell-containing cell culture gel is characterized in including: a step of adding cells and a coagulant to adult bovine blood-derived plasma and mixing to obtain a mixture; and a step of warming the mixture.

A method according to the present invention for producing a cell-containing cell culture gel is characterized in including: a step of adding a coagulant to adult bovine blood-derived plasma and mixing the same to obtain a mixture; a step of warming the mixture; a step of adding cells to the warmed mixture; and a step of further warming the cell-added mixture to bring about gelation thereof.

A method for producing cells according to the present invention is characterized in including: a step of carrying out cell culture using the aforementioned cell culture gel.

A method for producing cells according to the present invention also includes a step of carrying out cell culture using a cell-containing cell culture gel produced by the above-described method for producing a cell-containing cell culture gel.

Three-dimensional muscle tissue according to the present invention is produced by the above-described cell production methods.

Cultured meat according to the present invention is produced by the above-described cell production methods.

### ADVANTAGEOUS EFFECTS OF INVENTION

The cell culture gel according to the present invention is provided by using adult bovine blood-derived plasma and as a consequence can also be applied as a scaffold material during an edible cell culture, e.g., cultured meat.
In addition, the cell culture gel can be produced by adding an easily acquired coagulant, such as calcium chloride, to adult bovine blood (containing an anticoagulant) or adult bovine blood-derived plasma, which are distributed in relatively large quantities. As a consequence, the heretofore complex process for producing a scaffold material is simplified; in addition, the scaffold material purification process can also be omitted and the obtained scaffold material can be directly used in subsequent processes. As a consequence, utilization for cell culture in industrial production is also facilitated.
Moreover, an edible cultured meat exhibiting a higher safety is obtained by culturing muscle cells with a scaffold material that uses adult bovine blood-derived plasma.

### BRIEF DESCRIPTION OF DRAWINGS

[Fig. 1] Fig. 1 is a diagram that shows a summary of the collection of plasma and platelet-rich plasma.
[Fig. 2] Fig. 2 is a group of photographs that show the timewise changes (day 2, day 6, day 10) when myoblast cells are cultured using the cell culture gel.
[Fig. 3] Fig. 3 is a group of photomicrographs that show the contracted state of muscle tissue when electrical stimulation is applied to myoblast cells cultured in the cell culture gel.

### DESCRIPTION OF EMBODIMENTS

Embodiments of the present invention are specifically described in the following.

### (The cell culture gel)

The cell culture gel according to the present invention contains adult bovine blood-derived plasma and a coagulant.

In the present invention, "adult bovine" refers to a bovine or cattle that is not a fetus. Although the names and classifications of cattle by age vary depending on the specialized field, the adult bovines according to the present invention can be any age range for cattle after calving from a female bovine, such as very young cattle, young cattle, mature cattle, and old cattle. Mature cattle are more preferred from the standpoints of ready availability on the market and the number being slaughtered and processed.

The adult bovine blood can be obtained by a method in which a sample is collected from cattle that are slaughtered and processed at a meat market or wholesale marketplace, or can be obtained by other methods.

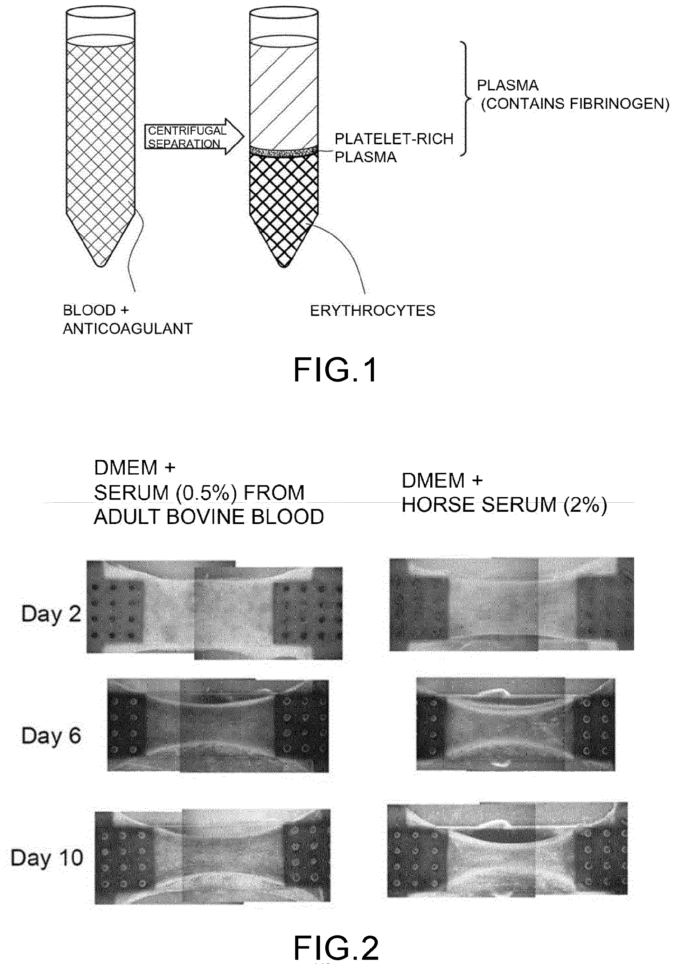

Adult bovine blood is typically shipped after the addition of an anticoagulant in order to prevent coagulation during distribution and storage. Anticoagulant-added adult bovine blood may be separated into erythrocytes and fibrinogen-containing plasma by centrifugation (see Fig. 1). Here, when an anticoagulant is added to adult bovine blood, neither the plasma containing fibrinogen, which becomes a coagulating component, nor the erythrocytes will coagulate or gel.

The plasma undergoes coagulation when a coagulant is added to the adult bovine blood-derived, fibrinogen-containing plasma yielded by centrifugal separation. The gelled plasma can be used as a cell culture scaffold material in three-dimensional and two-dimensional cell culture and is particularly suitable for three-dimensional cell culture. In the Description of the present application, this gelled plasma is defined as the cell culture gel.
Since the cell culture gel contains plasma, it is also expected to have the ability to replenish cell culture components that are nutrients for cell culture.

In addition, the aforementioned adult bovine blood-derived, fibrinogen-containing plasma may be a platelet-rich plasma (PRP). Platelet-rich plasma is a platelet-rich plasma concentrate produced by the centrifugal separation of plasma, and soluble protein and growth factors are concentrated therein in addition to platelets.

Platelet-rich plasma can be collected by subjecting anticoagulant-added adult bovine blood to, for example, centrifugal separation, double centrifugation separation, or selective filtration. Specifically, plasma from which the erythrocytes have been removed may be subjected to additional centrifugation and collection, or, for blood that also contains erythrocytes, the concentrated layer, within the plasma layer, at the boundary with the erythrocytes may be collected (see Fig. 1).

When platelet-rich plasma is used, gelation tends to progress more rapidly than for the use of whole plasma. Due to this, cell sedimentation is impeded and a more uniform cell dispersion can be provided using the cell culture gel that uses platelet-rich plasma.

The anticoagulant should be able to prevent blood coagulation, but is not otherwise limited, and can be exemplified by anticoagulants that bind the calcium ion, which is essential for blood coagulation, e.g., sodium citrate, ethylenediaminetetraacetic acid (EDTA), and sodium fluoride.
In the case of sodium citrate, it is desirably added, with reference to the total amount of the adult bovine blood and anticoagulant, at 2 volume% to 4 volume% and more preferably at around 3 volume%.

The coagulant should be able to gel the plasma from the anticoagulant-containing adult bovine blood, but is not otherwise limited. The coagulant can be exemplified by calcium chloride, Dulbecco's Modified Eagle's Medium (DMEM), and so forth, with calcium ion-containing coagulants being more preferred.

When the coagulant is calcium chloride, addition can be made so as to provide 5 mM to 70 mM, more preferably 10 mM to 65 mM, and still more preferably 10 mM to 60 mM as the calcium ion concentration with reference to the total amount of the plasma and coagulant.

DMEM contains L-arginine, L-cystine, L-histidine, L-isoleucine, L-leucine, L-lysine, L-phenylalanine, L-threonine, L-tryptophan, L-tyrosine, L-valine, calcium chloride, potassium chloride, magnesium sulfate, sodium chloride, sodium dihydrogen phosphate, D-glucose, folic acid, nicotinamide, riboflavin, vitamin B12, choline, inositol, pantothenic acid, pyridoxal phosphate, thiamine, iron, and so forth.
When the coagulant is DMEM, the adult bovine blood-derived plasma can be added in an amount that provides, relative to the amount of DMEM, at least 0.1 volume% and not more than 35 volume%, preferably at least 0.1 volume% and not more than 30 volume%, and more preferably at least 0.2 volume% and not more than 30 volume%.

The cell culture gel according to the present invention may additionally contain a culture media component besides the adult bovine blood-derived plasma and coagulant.

The culture media component can be exemplified by DMEM (e.g., GIBCO), EMEM (GIBCO), MEM ALPHA (GIBCO), RPMI-1640 (Roswell Park Memorial Institute 1640 culture medium; GIBCO), and so forth.
Moreover, additive components that are commonly used in culture media can be blended as appropriate in the culture media component. The additive components can be exemplified by antibiotics and also by vitamins, nucleic acids, amino acids, inorganic salts, sugars, polyamines, carbohydrates, proteins, fatty acids, lipids, pH modifiers, zinc, copper, selenium, and so forth.

### (Method for producing the cell-containing cell culture gel)

In one method according to the present invention for producing a cell-containing cell culture gel, cells and a coagulant are added to an adult bovine blood-derived plasma with mixing and the obtained mixture is warmed.

In another method according to the present invention for producing a cell-containing cell culture gel, a coagulant is added to adult bovine blood-derived plasma with mixing to obtain a mixture, the obtained mixture is warmed, cells are added to the warmed mixture, and gelation is carried out by further warming.

To culture cells in the above-described cell culture gel containing adult bovine blood-derived plasma and a coagulant, gelation is preferably performed prior to the cells becoming nonuniform due to, e.g., sedimentation. According to the present method for producing a cell-containing cell culture gel, the cells being cultured are uniformly dispersed in the cell culture gel and in addition gelation can be performed in a freely selected shape.

The cells to be cultured are not limited to a particular type as long as the cell culture gel containing adult bovine blood-derived plasma and coagulant can be utilized by the cells as a scaffold material.
Examples are primary culture cells, e.g., myoblast cells, tendon fibroblasts, and so forth; pluripotent stem cells, e.g., ES cells, iPS cells, and so forth, as well as adhesive cells that have been induced to differentiate from pluripotent stem cells; and established cell lines such as C2C12, CHO, HEK293, HL-60, HeLa, MDCK, NIH3T3, PC12, S2, Vero, and so forth.

Among the preceding, myoblast cells, upon proliferation and multinucleation, become skeletal myoblast cells having the form of myotubes (myotube cells) or muscle fibers. Myoblast cells can be exemplified by myoblast cells originating from vertebrates, e.g., mammals such as monkeys, cattle, horses, pigs, sheep, goats, dogs, cats, guinea pigs, rats, mice, and so forth; birds such as ostriches, chickens, ducks, sparrows, and so forth; reptiles such as snakes, crocodiles and alligators, lizards, turtles, and so forth; amphibians such as frogs, newts, salamanders, and so forth; and fish such as salmon, tuna, sharks, sea bream, carp, and so forth. In particular, when considering the possibility of having the proliferated and multinucleated skeletal myoblast cells be edible, myoblast cells derived from mammals raised as livestock, e.g., cattle, pigs, sheep, goats, and horses, are more desirable.

The suitable concentration of cells provided to the cell culture will vary depending on, for example, the composition of the culture medium and the concentration of the cell culture gel containing adult bovine blood-derived plasma and coagulant, but incorporation at a concentration that provides a proportion of 1 × 10⁶ cells/mL to 1 × 10⁸ cells/mL is preferred. The formation of a three-dimensional structure of the cells is impeded at low cell counts, while at high cell counts it is difficult to accommodate the cells within the cell culture gel.

The warming temperature range is preferably approximately 37°C. The warming time is adjusted in accordance with the state of progress of the gelation, and can be exemplified by approximately 5 minutes to 60 minutes and is more preferably approximately 10 minutes.

### (The cell production method)

The cell production method according to the present invention is a method of obtaining desired proliferated differentiated cells by culturing cells using the above-described cell culture gel containing adult bovine blood-derived plasma and coagulant or using the cell-containing cell culture gel described in the preceding.

Additional components as commonly used in cell culture may be present in the cell culture in addition to the adult bovine blood-derived plasma, coagulant, and cells.

This additional component can be exemplified by serum. The serum can be exemplified by adult bovine blood serum, horse serum, newborn calf serum, goat serum, rabbit serum, pig serum, chicken serum, and fetal bovine serum. Alternatively, the cell culture may be serum-free depending on the culture conditions. Among the preceding, adult bovine blood serum, horse serum, and pig serum are preferred from the standpoints of providing an edible product and the ease of securing quantities applicable to industrial production.

In an example of the cell production method, cells are mixed with the above-described adult bovine blood-derived plasma and the above-described coagulant, shaping is carried out by, for example, pouring into a form or mold with a freely selected shape, warming and gelation are carried out, and the cells are subsequently cultured.
In another example of the cell production method, the above-described adult bovine blood-derived plasma is mixed with the above-described coagulant, shaping is carried out by, for example, pouring into a form or mold with a freely selected shape, warming is carried out, cells are added, additional warming and gelation are carried out, and the cells are subsequently cultured.
With regard to the execution of cell culture, this can be exemplified by a method in which about 37°C is used for the culture temperature and a carbon dioxide concentration of 5% is used, but there is no limitation to this.

Besides the use of platelet-rich plasma as the plasma for the cell culture gel, when it is desired to improve the gelation rate preferably the cells and/or coagulant are mixed after the plasma has been preliminarily warmed at approximately 37°C.
An excessively fast or an excessively slow gelation rate is unfavorable. When gelation is excessively fast, gelation occurs prior to pouring into the form or mold for the gel and obtaining the desired shape is then impaired. When gelation is excessively slow, the cells sediment and a non-uniform cell-containing gel is ultimately provided.

When the cell culture gel is used for three-dimensional cell culture, the shape of the gel (i.e., the shape of the form or mold into which the mixture is poured prior to gelation) may be selected as appropriate depending on the application and objective. Examples are cylindrical shapes, rectangular parallelepiped shapes, thin plate shapes, ribbon shapes, spherical shapes, steak shapes, and so forth, as well as shapes having peaks and valleys or unevenness, shapes having gaps and/or spaces, and so forth.

In an example of a method for executing three-dimensional cell culture using myoblast cells or tendon fibroblasts, the gel is poured into a rectangular thin plate shape, as viewed from the top, on a support platform; both ends of the gel, considered in the longitudinal direction, are flowed into the gaps of a plurality of anchors; and cell culture is carried out. By fixing both ends of the gel with anchors, orientation of the muscle tissue in the longitudinal direction can be brought about because the contractile force of the muscle tissue acts as a tensioning force in the longitudinal direction.

### EXAMPLES

The present invention is more specifically described by the examples that follow. The following examples do not limit the scope of the present invention.

### (Evaluation test 1 of gelation of adult bovine blood-derived plasma)

Plasma derived from adult bovine blood (Tokyo Shibaura Zouki Co., Ltd.) that contained 3.13% sodium citrate as anticoagulant, was blended in different proportions with 100 volume% DMEM (GIBCO) and the presence/absence of gelation of the plasma was scored.

The results of the evaluation are given in Table 1.

**[Table 1]**

| | | | | | | | | | | |
|---|---|---|---|---|---|---|---|---|---|---|
| Proportion (volume%) of adult bovine blood-derived plasma relative to DMEM | 0.2 | 0.4 | 0.6 | 0.8 | 1 | 10 | 20 | 30 | 40 | 50 |
| Presence/absence of gelation of the plasma | Present | Present | Present | Present | Present | Present | Present | Moderately present | Absent | Absent |

The results in Table 1 demonstrated that, in the range of the evaluation in the Evaluation Test 1 of Gelation of Adult Bovine Blood-Derived Plasma, gelation of the adult bovine blood-derived plasma was observed when the plasma derived from sodium citrate-containing adult bovine blood was blended at 0.2 volume% to 30 volume% with 100 volume% DMEM.
Presumably the calcium ion required for blood coagulation caused gelation of the plasma as a consequence of a change in the equilibrium state between the sodium citrate and calcium ion upon the admixture into the plasma of the calcium chloride and other components present in the DMEM.

The preceding demonstrated that DMEM could cause the gelation of plasma derived from sodium citrate-containing adult bovine blood.

### (Evaluation test 2 of gelation of adult bovine blood-derived plasma)

The presence/absence of gelation of the plasma was scored when calcium chloride was blended at different concentrations into plasma derived from adult bovine blood (Tokyo Shibaura Zouki Co., Ltd.) that contained 3.13% sodium citrate as anticoagulant.

The results of the evaluation are given in Table 2.

**[Table 2]**

| | | | | | |
|---|---|---|---|---|---|
| Ca concentration (mM) | 12 | 24 | 60 | 90 | 120 |
| Presence/absence of gelation of the plasma | Present | Present | Present | Absent | Absent |

The results in Table 2 demonstrated that, in the range of the evaluation in the Evaluation Test 2 of Gelation of Adult Bovine Blood-Derived Plasma, gelation of the adult bovine blood-derived plasma was observed when calcium chloride was blended at 12 mM, 24 mM, and 60 mM as the Ca concentration into plasma from sodium citrate-containing adult bovine blood. It was found in particular that gelation of the plasma advanced further when the Ca concentration was 24 mM.
Presumably the calcium ion required for blood coagulation caused gelation of the plasma as a consequence of a change in the equilibrium state between the sodium citrate and calcium ion upon the admixture of calcium chloride into the plasma.

The preceding demonstrated that calcium chloride could cause the gelation of plasma derived from sodium citrate-containing adult bovine blood.

### (Cell culture test)

Adult bovine blood (Tokyo Shibaura Zouki Co., Ltd.) containing 3.13% sodium citrate as anticoagulant was subjected to centrifugal separation and the plasma was collected.

Rat myoblast cells (1 × 10⁸ cells/mL) were blended into a mixture of calcium chloride (20 mM as the Ca concentration) and plasma that had been preliminarily warmed to about 37°C.

This pre-gelation mixture was poured into a three-dimensional tissue forming tool and was warmed at 37°C to induce gelation, and culture medium was subsequently added and cell culture was carried out. The gel shape formed by the three-dimensional tissue forming tool has a rectangular thin plate shape, as viewed from the top, and both ends, considered in the longitudinal direction, are fixed by a plurality of anchors. The poured-in size of the gel on the inner side, excluding the anchors, was 0.4 cm × 0.5 cm. Culture was carried out for 2 days in a culture medium containing, as a culture media component, DMEM containing 10% adult bovine blood serum, followed by culture for an additional 8 days in the culture medium blended with DMEM containing 0.5% adult bovine blood serum or DMEM containing 2% horse serum. The cultured cells were submitted to microscopic observation on day 2, day 6, and day 10. In addition, electrical stimulation (frequency: 1 Hz, strength: 1.5 V/mm, duration: 20 ms) was applied to the cells and the state of contraction was observed with a microscope.

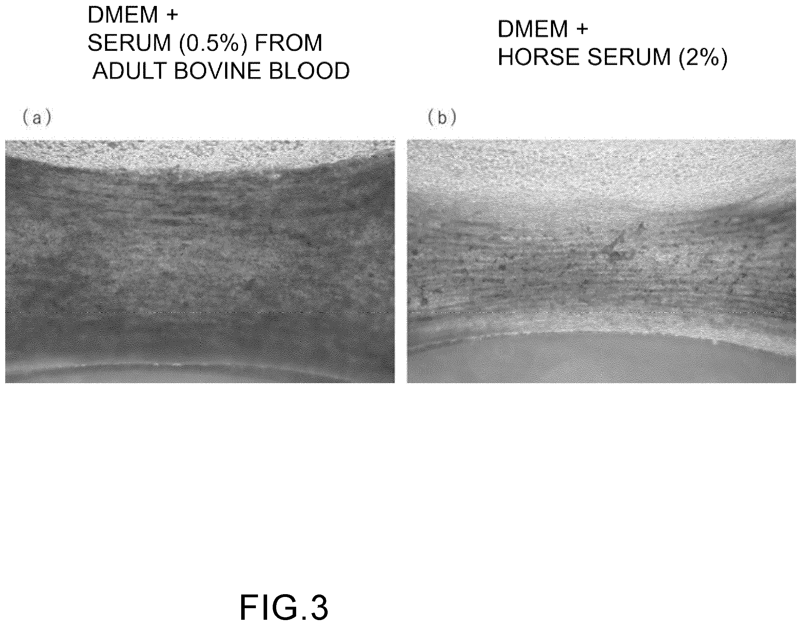

The results of the cell culture test are given in Figs. 2 and 3.

According to Fig. 2, it was found that a three-dimensional cell culture was possible when a cell culture gel containing adult bovine blood-derived plasma and coagulant was used as a scaffold material. The formation of tissue having a three-dimensional structure was similarly confirmed when adult bovine blood serum and when horse serum were used as the serum.

For the three-dimensional muscle tissue obtained by culturing using, as a scaffold material, a cell culture gel containing a coagulant and plasma from adult bovine blood, it was confirmed in accordance with Fig. 3 that contraction occurred under electrical stimulation and that differentiation had progressed.

It was demonstrated, in accordance with the preceding, that the cell culture gel containing adult bovine blood-derived plasma and coagulant can bring about the proliferation and differentiation of myoblast cells in the same manner as extracellular matrix.

## Claims

1. A cell culture gel comprising:
adult bovine blood-derived plasma; and
a coagulant.

2. The cell culture gel according to claim 1, wherein the adult bovine blood-derived plasma is a platelet-rich plasma.

3. The cell culture gel according to claim 1 or 2, wherein the coagulant is calcium chloride.

4. The cell culture gel according to any one of claims 1 to 3, further comprising a culture media component.

5. A method for producing a cell-containing cell culture gel, the method comprising:
a step of adding cells and a coagulant to adult bovine blood-derived plasma and mixing the same to obtain a mixture; and
a step of warming the mixture.

6. A method for producing a cell-containing cell culture gel, the method comprising: a step of adding a coagulant to adult bovine blood-derived plasma and mixing the same to obtain a mixture;
a step of warming the mixture;
a step of adding cells to the warmed mixture; and
a step of further warming the cell-added mixture to bring about gelation thereof.

7. A method for producing cells, comprising:
a step of carrying out cell culture using the cell culture gel according to any one of claims 1 to 4.

8. A method for producing cells, the method comprising:
a step of carrying out cell culture using a cell-containing cell culture gel produced by the method for producing a cell-containing cell culture gel according to claim 5 or 6.

9. A three-dimensional muscle tissue produced by the cell production method according to claim 7 or 8.

10. A cultured meat produced by the cell production method according to claim 7 or 8.
